# EUROPEAN PATENT APPLICATION

(11) **EP 4 744 616 A2**
(43) Date of publication of application: **20.05.2026**
(21) Application number: 26163039.6
(22) Date of filing: 23.06.2020
(51) Int. Cl.: A61C 1/18

(54) **DENTAL SCREW AND DENTAL FASTENING TOOL**

(30) Priority: 26.06.2019 EP 19182620
(62) Divisional of application: 20733638.9
(71) Applicant: Nobel Biocare Services AG, 8302 Kloten (CH)
(72) Inventor: Nagel, Patrick, 8105 Regensdorf (CH); Christiansen, Piers, 8003 Zürich (CH); Ettlin, Karin, 5430 Wettingen (CH)
(74) Representative: Jensen, Olaf Sven

(57) **Abstract**

The present disclosure provides a dental screw (10) for fixing dental components (1, 2) to each other, the dental screw having a proximal end (11), a distal end (12), and a longitudinal axis (LS), wherein the dental screw further comprises a screw head (13) including a tool engaging interface (20) for engaging a fastening tool (30) at the proximal end and distally to the screw head a screw shank (14), the screw shank comprising a torque limiting portion (15) and distally to the torque limiting portion a threaded portion (16) for engaging a dental component (1, 2, 3), wherein the dental screw is configured to transfer a minimum torque from the tool engaging interface to the threaded portion of 26 Ncm and the torque limiting portion is configured to limit the torque transferable to a maximum torque for preventing damage to the threaded portion. The disclosure also provides a dental fastening tool, a dental fastening set, and a dental support set.

## Description

### TECHNICAL FIELD

The present invention relates to a dental screw, a dental fastening tool, a dental fastening set, and a dental restoration support set.

### BACKGROUND OF THE INVENTION

A dental restoration that is anchored within the mouth of a patient using a dental implant has been proven to be a very successful solution for the replacement of one or more teeth. By now, there are numerous techniques for dental treatments available that allow for an aesthetical integration of a dental restoration into the dentition of a patient. Further, these dental restorations are also from a functional viewpoint basically identical to the one or more native teeth they replace so that patients do not experience major changes in comparison to a healthy native counterpart.

In order to be able to offer such a solution of a dental restoration to patients in a customized and economical manner, dental restorations generally have a modular design. Thus, these dental restorations are adapted to an individual patient by choosing a combination of dental components that provides the desired result for the replacement. A combination of dental components may simply be a dental implant that is directly connected to an artificial tooth. More commonly, it is a combination of an abutment, a dental implant, and an artificial tooth, wherein the abutment is arranged between the dental implant and the artificial tooth. Such abutments are available as one-piece abutments and multi-piece abutments. Further components that can be present in a dental restoration are bridging elements, caps, etc.

One of the most common techniques to assemble a combination of dental components is the use of dental screws. Dental screws allow for both assembly and disassembly of a dental restoration. This is particularly advantageous for adapting a dental restoration in a dental lab as well as at the chair site in a dental clinic. Further, the use of screws renders a revision of at least parts of a dental restoration much easier than in case of for example using dental cement for connecting these components. Such dental screws have been proposed in WO 2018/091515 A1, WO 2017/070335 A1, and US 8,029,282 B2.

However, an increasing number of components requires a smaller size of dental screws in order to optimize adaptability of the dental restoration. Smaller dental screws also provide more flexibility in an area of a dentition where space is rather limited such as the front teeth.

These dental screws are used to assemble and fixate the dental components in relation to each other, wherein masticatory forces are primarily transferred through the dental components. Consequently, the primary function of the dental screws is to keep the assembly mounted. Further, the smaller the dental screws, the more material can be designated for the dental components to increase their strength for withstanding masticatory forces.

### SUMMARY OF THE INVENTION

In view of the above, one of the objectives of the present disclosure was to provide dental screws with a design that allows for a further reduction of their size. However, the smaller the screws, the harder the handling of these screws becomes. As a result, another objective of the present disclosure was to enhance handling of such small dental screws. Further, a reduced size of these dental screws bears the risk to cause damage to the screws or dental components, in particular when transferring torque during tightening of the screws. Consequently, it was yet another objective of the present disclosure to improve the transfer of torque between fastening tools, screws, and/or dental components. The inventors also had a cost-efficient manufacturing of these dental screws, drivers, and dental components in mind.

In order to address these objectives, the present disclosure provides a dental screw for fixing dental components to each other. The dental screw has a proximal end, a distal end, and a longitudinal axis. The dental screw further comprises a screw head including a tool engaging interface for engaging a fastening tool at the proximal end and distally to the screw head a screw shank. The screw shank comprises a torque limiting portion and distally to the torque limiting portion a threaded portion for engaging a dental component. The dental screw is configured to transfer a minimum torque of 26 Ncm and the torque limiting portion is configured to limit the torque transferable to a predetermined maximum torque, in particular at a maximum tensile and or shear stress, for preventing damage to the threaded portion.

The torque limiting portion implemented as part of the screw shank causes the screw shank to fail if the maximum torque, in particular at a maximum tensile and/or shear stress, is exceeded. In other words, the material of the dental screw at the torque limiting portion is not able to withstand the shear forces caused by a torque that exceeds the maximum torque and that is applied via the tool engaging interface. However, the dental screw is configured to transfer a minimum torque of 26 Ncm in order to be able to securely fasten dental components to each other.

As a result, the torque limiting portion prevents damage to the threaded portion of the dental screw as well as to the threaded portion of the dental component the dental screw is engaged with. For example, the dental component may be a dental implant so that the dental screw prevents damage to the implant. Consequently, it also provides as a benefit to a patient that this configuration of a dental screw prevents a revision of the implant due to such a damage.

Generally, the dental screw is in threaded engagement with a first dental component in order to fasten a second dental component to this first dental component. Resulting from the predefined failure of the dental screw at the torque limiting portion, the second dental component that the dental screw is intended to fasten may easily be removed. Further, the section of the shank remaining engaged with the first dental component after failure can also be easily removed using a tool such as pliers. As a result, the first dental component remains intact and does not have to be replaced. In fact, the only part that has to be replaced is the dental screw.

The torque limiting portion is preferably arranged (in a portion) adjacent to the screw head so that after failure of the torque limiting portion the major part of the shank engaged with the dental component remains in the dental component the dental screw was engaged with before failure. This facilitates the removal of the residual shank of the dental screw from this dental component. In other words, the torque limiting portion is preferably in a coronal (proximal) portion of the screw.

Due to the presence of a torque limiting portion, it is further possible to reduce the size of the dental screw without risking failure of the dental screw at a section of the dental screw that may cause damage to a dental component.

For weakening the dental screw at the torque limiting portion, the torque limiting portion may have the smallest section modulus along the shank.

Thus, the cross-section of the torque limiting portion is designed to have the least resistance to torque and, thus, breaks first if a torque is applied that exceeds the maximum torque the torque limiting portion is designed to transfer. In other words, the section modulus of the torque limiting portion is smaller than the section modulus of the remaining shank including the threaded portion. The section modulus is preferably also smaller than any cross-section of the screw head that is designed for transferring a torque applied via the tool engaging interface.

Alternatively or additionally, the torque limiting portion may comprise at least one notch for initiating failure, may be made of a different material and/or may have different material properties.

Preferably, the maximum torque transferable at the torque limiting portion is 38 Ncm, 36 Ncm, 34 Ncm, or 32 Ncm. Applying a torque beyond the torque value chosen for the design of the dental screw will result in failure of the torque limiting portion and, thus, in failure of the dental screw.

Further, the torque limiting portion may be configured to withstand a maximum tensile force in a range of 300 N to 500 N and preferably in a range of 350 N to 450 N.

The application of torque results in a tensile force that is acting along the screw shank between the head of the screw and the threaded portion, i.e. also along the torque limiting portion. Exceeding the maximum tensile force a dental screw is designed for, in particular during application of torque, is configured to cause rupture of the screw shank at the torque limiting portion and, thus, failure of the dental screw.

The tool engaging interface may include a torque transfer zone that has a cross-section perpendicular to the longitudinal axis of the dental screw comprising a profile with four or five convex and/or planar sections alternating with four or five concave sections, respectively.

These concave and convex sections form an undulating profile about the longitudinal axis of the dental screw. As a result, the transfer of torque via the tool engaging interface is enhanced. This is primarily caused by the torque being transferred across contact surfaces extending in a more radial direction between a corresponding dental fastening tool and the dental screw. Further, no force in the direction of the dental screw's longitudinal axis has to be applied for keeping the fastening tool and the dental screw engaged during the application of torque for fastening the dental screw to a dental component.

Further, having four or five convex and/or planar sections that alternate with four or five concave sections enables a design with a smaller screw head with sufficiently thick walls for transferring a predetermined torque and at the same time allowing for a compact design of the screw. It also allows for a screw driver with a smaller diameter. A higher number of sections requires the screw driver to be bigger, which results in an increase in size of the screw head and/or thinner walls that weaken the screw. The tool engaging interface is preferably formed as a recess.

It has been found that a higher number of concave sections and convex and/or planar sections primarily weakens the screw head, whereas a smaller number of concave and convex/planar sections shows an adverse effect in relation to torque transfer.

Preferably, the convex and/or planar sections and concave sections are connected or are merged at points of transition, and at each point of transition an angle between a tangent to the profile of the concave section and a tangent to the convex section is in a range of 90° to 180° or to 160°, preferably 95° to 150°.

Implementing an angle for a point of transition in the design of the dental screw that lies within above-listed ranges has been shown to provide for a smooth and efficient transfer of torque.

The point of transition between the alternating concave sections and convex and/or planar sections may be a point along the profile of the tool engaging interface, where two alternating sections continuously or smoothly transition from one to the other. In this case, the point of transition represents the point where the curvature changes signs with a continuous change in slope. As a result, a tangent to this point of transition has the same slope when being based on the adjacent concave section or the adjacent convex and/or planar section.

Alternatively, the point of transition may also be a point, where the alternating sections transition from one to the other in a discontinuous manner. In this case, the curvature changes signs or its direction with a sudden shift in slope. Consequently, a tangent at this point of transition derived based on the adjacent concave section results in a different slope compared to a tangent at this point of transition derived based on the adjacent convex and/or planar section.

The convex and/or planar sections and concave sections may form longitudinal engagement protrusions radially extending from a circle with a maximum inner diameter towards the longitudinal axis. The engagement protrusions preferably have chamfers at their proximal ends.

These longitudinal engagement protrusions are configured for engaging a fastening tool and receiving torque from this tool. In other words, they form at least a part of the torque transfer zone. If chamfers are present at the proximal ends of the engagement protrusions, it is easier for a dental professional to bring a fastening tool into engagement with the dental screw. More specifically, the chamfers that are preferably situated at the entrance of the tool engaging interface guide a fastening tool into engagement.

The tool engaging interface may include a pick-up zone that tapers in a direction from the proximal end to the distal end of the dental screw. The pick-up zone is preferably located adjacent, proximally to, and/or at least partially along the torque transfer zone.

The taper of the pick-up zone allows for retaining the dental screw to a dental fastening tool via friction forces. In other words, the dental fastening tool may be inserted into the pick-up zone. Then, sufficient pressure is applied in order to press the dental fastening tool into the pick-up zone. This establishes a friction fit that can be easily reversed. For this reversible friction fit, the angle of the taper is preferably chosen in a range of 5° to 12°, in particular 6° to 9°, or substantially 7°. Alternatively, it may be in a range of 10° to 12° or substantially 11°.

Particularly if the pick-up zone extends at least partially along the torque transfer zone, i. e. the torque transfer zone and the pick-up zone are at least partially overlapping or identical, the tapered configuration results in the advantage of a continuously efficient torque transfer in the presence of wear. More specifically, if the dental fastening tool and/or the tool engaging interface of the dental screw are subject to wear, the dental fastening tool will simply be inserted further into the tapered torque transfer zone. Thus, torque transfer is essential not altered since the structure, where the torque transfer takes place, basically remains the same despite from wear occurring.

On the one hand, if the torque transfer zone and the pick-up zone at least partially overlap, this allows a more compact design of the screw head and the fastening tool along their longitudinal axes. On the other hand, if the pick-up zone and torque transfer zone do no overlap, wear may be reduced and a more precise fit of dental screw and fastening tool may be achieved due to the functional and spatial separation of the two zones.

The tool engaging interface may further include a verification zone for verifying the type of the dental screw. The verification zone is preferably located at the distal end of the tool engaging interface.

Based on the verification zone, a dental professional is in a position to verify if the dental screw is the appropriate screw to be used with a dental fastening tool or a dental component. For example, if a dental professional is not able to fully insert into and/or engage the fastening tool with the tool engaging interface, this indicates that either an incorrect screw or an incorrect dental component has been chosen for assembly. Consequently, such a verification zone prevents damage to a dental component or a dental screw if their configurations do not complement each other.

Alternatively or additionally to a verification zone, an appropriate combination of a dental component and a dental screw may also be ensured by configuring these two dental components so that they can only be assembled using a dental screw of correctly positioned and oriented in relation to each other. This is particularly described in detail in relation to figures 5 in the international application also filed by the present applicant with the number PCT/EP2018/071797, which is hereby incorporated by reference.

Further, the disclosure provides a dental fastening tool. The dental fastening tool has a proximal end, a distal end, and a longitudinal axis. The dental fastening tool comprises a screw engaging interface at the distal end for engaging a dental screw, in particular a dental screw as described above, and proximally to the screw engaging interface a torque limiting portion. The torque limiting portion is configured to limit the torque transferable to a maximum torque for preventing damage to the screw engaging interface.

The advantage of a dental fastening tool comprising a torque limiting portion is similar to the above described advantages achieved with a torque limiting portion incorporated into a dental screw. More specifically, if the torque transferred by the dental fastening tool exceeds the predefined maximum torque, the torque limiting portion is configured to fail. This failure prevents damage to the dental screw. After failure, the parts of the dental fastening tool separated at the torque limiting portion can easily be removed so that another dental fastening tool may be used to finish the assembly or to remove the dental screw, for example for checking the cause of the failure. In any case, the torque limiting portion of the dental fastening tool keeps damage from occurring at the level of a dental component, such as a dental implant or an abutment.

As already discussed above, the torque limiting portion also allows for a smaller design of the dental screw. Safety factors that are normally included for preventing damage to or failure of the dental screw or a dental component to be engaged with or fastened to the dental screw can be reduced. Instead of trying to prevent any damage, damage is controlled by a predetermined failure of the dental screw. In other words, the safety provided by means of safety margins is at least partly replaced by the safety mechanism of a controlled failure.

The torque limiting portion may be configured to bear a maximum torque of 35 Ncm or less, preferably in a range of 27.5 Ncm to 34 Ncm and more preferably in a range of 28 Ncm to 33 Ncm.

The higher values of these ranges such as 30 to 34 Ncm are preferably used for a dental fastening tool that also allows to transfer torque while the longitudinal axis of the dental fastening tool and the longitudinal axis of the dental screw are inclined in relation to each other. The lower values of these ranges, such as 27.5 to 29 Ncm are sufficient for dental fastening tools that do not allow for such an inclination during a transfer of torque.

The screw engaging interface may include a torque transfer zone with a cross-section perpendicular to the longitudinal axis comprising four or five convex and/or planar sections alternating with four or five concave sections. The convex and/or planar sections and concave sections form an engagement structure about the longitudinal axis along the dental fastening tool.

The advantages of such a configuration of the torque transfer zone has already been discussed in relation to the corresponding zone of the dental screw. In terms of the dental fastening tool it should be noted that it is particularly advantageous to use five concave sections alternating with five convex and/or planar sections if the dental fastening tool is designed for a transfer of torque also in the aforementioned angled relationship to the dental screw.

A dental fastening tool that allows for an angled torque transfer between the longitudinal axis of the dental fastening tool and the longitudinal axis of a dental screw preferably has a curved profile in a cross-section along the longitudinal axis (longitudinal cross-section).

The curved profile preferably extends over an angle in this longitudinal cross-section so that it allows for a torque transfer taking place between the dental fastening tool and the dental screw while the relative angle between their longitudinal axes is in a range of 0 to 25°. Consequently, the curved profile may extend over a range of at least 25°.

The concave sections preferably have a curved profile in a cross-section perpendicular to the longitudinal axis of the dental fastening tool. Further, the convex sections may have a curved profile, in particular a circular shaped profile, or may include a profile that is both curved and straight (linear). This corresponds to a convex and planar section along the circumference of the screw engaging interface. As previously described, it is also possible to use a planar section instead of a convex section. The skilled person will appreciate that a planar section is represented by a straight line in the fastening tool's cross-section perpendicular to the longitudinal axis.

Preferably, the convex and/or planar sections and concave sections are connected or are merged at points of transition, and at each point of transition an angle between a tangent to the profile of the concave section and a tangent to the convex section is in a range of 90° to 180° or to 160°, preferably 95° to 150°.

Similar to the dental screw, implementing an angle for a point of transition in the design of the dental fastening tool that lies within above-listed ranges has been shown to provide for a smooth and efficient transfer of torque.

The lower angles in a range of 90° to 110° and in particular 96° to 104° particularly enhance torque transfer between the tool engaging interface of a dental screw and a screw engaging interface of a dental fastening tool.

The greater angles in a range of 130° to 180° and in particular in a range of 142° to 148° may advantageously be combined with a higher number of aforenoted concave sections and convex and/or planar sections, in particular five of each. The angles in these ranges still provide for an efficient transfer of torque. They further allow for a configuration of the screw engaging interface that is able to transfer torque while the longitudinal tool axis and the longitudinal screw axis are not aligned, i. e. are inclined in relation to each other.

The angle at the point of transition of the dental screw's profile preferably substantially matches the angle at the point of transition of the dental fastening tool.

The point of transition between the alternating concave sections and convex and/or planar sections may be a point along the profile of the screw engaging interface, where two alternating sections continuously or smoothly transition from one to the other. In this case, the point of transition represents the point where the curvature changes signs with a continuous change in slope. As a result, a tangent to this point of transition has the same slope when being based on the adjacent concave section or the adjacent convex and/or planar section.

Alternatively, the point of transition may also be a point, where the alternating sections transition from one to the other in a discontinuous manner. In this case, the curvature changes signs or its direction with a sudden shift in slope. Consequently, a tangent at this point of transition derived based on the adjacent concave section results in a different slope compared to a tangent at this point of transition derived based on the adjacent convex and/or planar section.

The screw engaging interface may include a pick-up zone for holding a dental screw, the pick-up zone preferably being located adjacent, proximally to, and/or at least partially along the torque transfer zone.

The presence of a pick-up zone facilitates handling of a dental screw since the dental screw can be handled while being attached to the dental fastening tool. This is also advantageous since it prevents contamination of the dental screw during handling.

The pick-up zone may have a conical shape, in particular if the transfer of torque can only be effected while the longitudinal axis of a dental screw and the dental fastening tool are aligned with each other.

Alternatively, the pick-up zone may also have a curved profile in the longitudinal cross-section as described above in relation to the curved torque transfer zone of the dental fastening tool. Such a pick-up zone may also at least partly serve as a torque transfer zone.

In any case, the pick-up zone will, for example, be able to interact with a dental screw having a tapering tool engaging interface in order to establish the pick-up and retaining function via friction.

The screw engaging interface may further include a verification zone for verifying the type of a dental screw when engaging said dental screw. The verification zone is preferably located at the distal end of the tool engaging interface.

As described in relation to the dental screw from above, the verification zone has the purpose to verify an appropriate use of the dental fastening tool, e. g. that the dental fastening tool is used with a corresponding dental screw. Thus, it is possible to prevent an unintended use of the dental fastening tool.

The disclosure further provides a dental fastening set. The dental fastening set comprises a dental screw, in particular a dental screw according to one of the previously described configurations. The dental fastening set also comprises a dental fastening tool and in particular a dental fastening tool configured as described above. The dental screw and the dental fastening tool each comprise a torque limiting portion, wherein the torque limiting portion of the dental screw is configured to bear a maximum torque that is higher than the maximum torque that is transferable via the torque limiting portion of the dental fastening tool.

For a transfer of torque, the dental fastening tool and the dental screw correspond to each other. More specifically, the tool engaging interface of the screw and the screw engaging interface of the dental fastening tool correspond to each other.

Due to above-defined relationship between the torque limiting portion of the dental fastening tool and the torque limiting portion of the dental screw, failure during use of the dental screw and the dental fastening tool is configured to occur on the side of the fastening tool first. Consequently, damage to the dental screw or dental components is prevented. Designing the fastening set so that failure occurs further away from the dental component that is in engagement with the dental screw (such as a dental implant) makes the replacement of any of these dental components, dental screws or dental tools easier.

The dental screw of the dental fastening set may comprise a tool engaging interface, wherein the tool engaging interface includes a torque transfer zone with a cross-section comprising convex and/or planar sections alternating with concave sections, wherein the convex and concave sections preferably have curved profiles. For transferring torque in an engaged state of the dental screw and the dental fastening tool, at least an intermediate section of each of the convex and/or planar sections of the dental screw faces a profile of a concave section of the tool engaging interface at a distance.

Due to this distance, the convex and/or planar section of the dental fastening tool is not in engagement with the dental screw. This prevents a transfer of torque from the dental fastening tool to the dental screw at the part of the profile that would be otherwise rather inefficient. The transfer of torque may also cause an increase in wear due to the orientation of the contacting surface that negatively affects the transfer of torque as will be described in more detail further below. In other words, with such a configuration the torque transfer primarily takes place at the points of transition between the concave section and the convex and/or planar section of the screw engaging interface of the dental fastening tool and the tool engaging interface of the dental screw.

This results in this configuration also reducing wear at the convex and/or planar sections of the dental fastening tool. Wear may be reduced even further by using steel as a material for the dental fastening tool and, preferably, titanium or a titanium alloy as a material for the dental screw.

The torque transfer zone of the dental screw and the torque transfer zone of the dental fastening tool have a rotational play when being engaged with each other, in particular a play in a range of 1° to 3.5° and preferably 1.5° to 3°.

In other words, the cross-sectional profile of the torque transfer zone of the dental screw and the torque transfer zone of the dental fastening tool correspond to each other but do not necessarily match each other completely so that they are configured to provide such a rotational play. This rotational play serves as haptic feedback to a dental professional for determining the begin of a torque transfer. In particular, the dental professional is able to easily realize when contact is established between the profile of the dental fastening tool and the dental screw during rotation and, thus, the initiation of torque transfer. The rotational play also facilitates insertion of the dental fastening tool. This particularly applies if the dental fastening tool is configured to (only) allow torque transfer with the longitudinal axes of the dental fastening tool and the dental screw being aligned with each other.

The dental screw and the dental fastening tool may further each comprise a pick-up zone configured to engage each other by friction. As already described above, such a pick-up zone facilitates handling of the dental screw and prevents contamination, in particular since a dental professional does not have to directly hold the dental screw.

The dental screw and the dental fastening tool may further each comprise a verification zone configured to allow engagement of the torque transfer zone of the dental fastening tool and the torque transfer zone of the dental screw when the dental fastening set is fully engaged.

In other words, a transfer of torque is only truly enabled, if the verification zones of the dental screw and the dental fastening tool are in full engagement. Without full engagement, the transfer of torque is at least hardly achievable and preferably impossible. As discussed above, this prevents damage to the dental fastening tool, the dental screw, and dental components from occuring of these are not intended to be used in combination.

Further, the disclosure provides a dental restoration support set comprising a dental screw, in particular a dental screw according to a configuration described above, and a dental component, wherein the dental component is in particular a dental implant, an abutment, and/or an artificial tooth.

In this combination, the smaller design of the dental screw allows for an increase in strength of the dental restoration support set and/or a smaller size of the dental restoration support set.

The dental screw may comprise a support surface. The support surface may be inclined in relation to a longitudinal axis of the dental component. Accordingly, the dental component comprises a screw seat. The screw seat may be inclined at an angle in relation to the longitudinal axis of the dental screw so that at least in a pre-fastened state a contact between the support surface of the dental screw and the screw seat is generally established by a line contact. During contact of the screw seat and the support surface, the longitudinal axis of the screw seat is aligned with the longitudinal axis of the support surface.

The angular relationship between the support surface of the dental screw and the screw seat of the dental component that at least initially results in a line contact between the support surface and the screw seat has the advantage that this contact is more predictable than a surface contact leading to a better predictability of the fastening process. Consequently, it helps in predetermining the flow of forces within the screw and, thus, improves the predictability of failure. The line contact is preferably at a position at or close to the outer diameter of the screw head.

When being fastened, the angular gap between the support surface of the dental screw and the screw seat of the dental component may at least partially close. As a result, the increase in torque is dampened at the end of fastening. The dental professional can predict the end of fastening more easily. As a result, it is less likely that damage to the dental screw, the dental fastening tool, or the dental component occurs.

The screw seat as well as the support surface are preferably both formed as conical angular surfaces.

The dental screw comprises a threaded portion and the dental component comprises a threaded hole. In a fastened state, the thread flank of the threaded portion and the thread flank of the threaded hole, which are in contact with each other, are each in contact along at least 70% of their profile's length in a radial direction.

In other words, when looking at the contact between the two flanks of the threads of the dental screw and the dental component in a profile of a longitudinal cross-section of the screw in a fastened state, the two thread profiles are in contact with each other along a length of at least 70% of each of their lengths. More specifically, the proximal flank of the dental screw's threaded portion is in contact with a distal flank of a dental component's threaded hole along at least 70% of each of their lengths along the thread flanks in a radial direction in relation to the dental screw's longitudinal axis.

This configuration has the advantage that the surface contact between the dental screw and the dental component is enhanced compared to normal threads, such as metric ISO-threads, so that the distribution of forces for fastening the dental screw within the threaded hole of the dental component is more uniform. It also allows for a build-up of the necessary tensile force for fastening with fewer revolutions and, thus, a quicker fastening and enables a shorter thread design. This enables a smaller design for the dental screw as well as for the threaded hole of the dental component. The strength of the dental restoration support set can be increased and/or its total size can be decreased.

### SHORT DESCRIPTION OF THE DRAWINGS

The following figures illustrate preferred embodiments of the present invention. These embodiments are not to be construed as limiting but merely for enhancing the understanding of the invention in context with the following description. In these figures, same reference signs refer to features throughout the drawings that have the same or an equivalent function and/or structure. A repetitive description of these features is generally omitted for a concise description.
Figure 1 is a cross-sectional view along the longitudinal axis of a dental screw and in particular of a tool engaging interface of the dental screw;
Figure 2 is a plan view of the tool engaging interface of the dental screw illustrated in figure 1;
Figures 3a and 3b are cross-sectional views perpendicular to the longitudinal axis of the dental screw shown in figures 1 and 2 illustrating the tool engaging interface in engagement with a screw engaging interface of a dental fastening tool;
Figure 4 is a partial side view of the dental fastening tool shown in figures 3a and 3b;
Figure 5 is a cross-sectional view perpendicular to the longitudinal axis of the dental fastening tool shown in figures 3 and 4 at the level of a torque transmission section of the fastening tool;
Figures 6a and 6b are cross-sectional views along the longitudinal axes of the dental screws at the level of the tool engaging interface and in engagement with a dental fastening tool;
Figure 7 is a three-dimensional view of a dental screw according to another embodiment;
Figure 8 is a three-dimensional view of a dental fastening tool according to another embodiment for fastening the dental screw shown in figure 7;
Figure 9 is a detailed view of the screw engaging interface of the dental fastening tool of figure 8;
Figures 10a and 10b illustrate the functionality of the screw engaging interface's verification zone;
Figure 11 is a side view of another embodiment of a screw engaging interface of a dental fastening tool;
Figure 12 is a frontal view of the screw engaging interface of the dental fastening tool shown in figure 11;
Figure 13 is a schematic cross-sectional view of a threaded engagement between a dental screw and a dental implant; and
Figure 14 is a cross-sectional view along the longitudinal axes of a dental screw, an abutment, and an implant in engagement according to yet another embodiment.

### DETAILED DESCRIPTION OF PREFERRED EMBODIMENTS

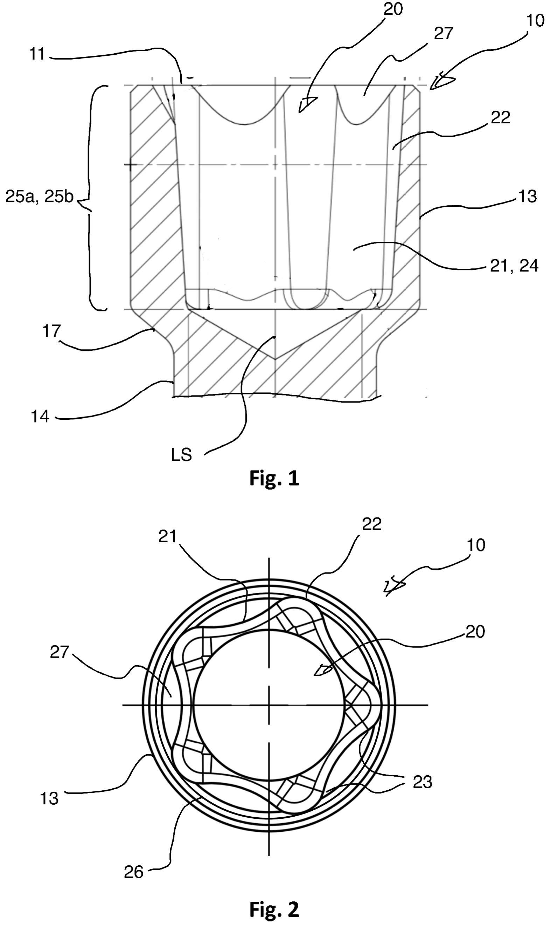

Figure 1 shows a cross-sectional view along the longitudinal axis LS of a dental screw 10 according to an embodiment. In the following, a cross-section along a longitudinal axis is also referred to as a longitudinal cross-section. The longitudinal cross-section of the dental screw 10 in figure 1 shows the screw head 13 and a part of the screw shank 14. The screw head 13 comprises a tool engaging interface 20. As shown in figure 1, the tool engaging interface 20 is preferably formed as a recess.

For transferring torque, the tool engaging interface 20 comprises an engagement structure. This engagement structure includes convex sections 21 and concave sections 22. The convex sections 21 alternate with the concave sections 22. There is an equal number of convex sections 21 and concave sections 22. As illustrated, these sections are arranged on an inner circumferential wall of the tool engaging interface 20 and form a torque transfer zone 25b.

The tool engaging interface 20 of the exemplary embodiment of a dental screw in figure 2 comprises five convex sections 21 and five concave sections 22. As seen in a cross-section perpendicular to the longitudinal axis LS of the dental screw 10 (also referred to in the following as a perpendicular cross-section), the convex sections 21 and concave sections 22 form longitudinal engagement protrusions 24 extending from a circle 26. This circle is defined by the maximum inner diameter of the tool engaging interface 20. In case of a tapered tool engaging interface 20 as further described below, the diameter of the circle 26 decreases from the proximal end to the distal end of the dental screw 10.

In order to achieve a compact yet durable design for the dental screw 10, it has been found to be advantageous to provide the tool engaging interface 20 with four or five concave and convex sections each. Preferably, the screw engaging interface of a dental fastening tool comprises numbers of concave and convex sections that corresponds to the numbers of the concave and convex sections of a dental screw to be engaged.

As will be described in more detail below, five sections of each of the convex and concave sections is preferred for a fastening tool that allows an efficient and an angular application of torque (i. e. an application of torque via an angle).

A number of four sections of each of the concave and convex sections has been found to be particularly advantageous for a combination of a dental screw and a fastening tool whose longitudinal axes are aligned during the application of torque. In such an embodiment, the geometry of the engagement structures results in a very precise engagement and efficient torque transfer.

In the exemplary embodiments of dental screws and fastening tools shown in the figures, the concave sections 22 and the convex sections 21 have a curved profile. Nonetheless and as previously described, the convex sections 21 may also be planar. The concave sections 22 and convex sections 21 form an engagement structure. Along the profile of the engagement structure in a perpendicular cross-section, the concave sections 22 and the convex sections 21 are connected at points of transition 23. In the embodiment of figure 2, the transition between the concave sections 22 and the convex sections 21 is continuous.

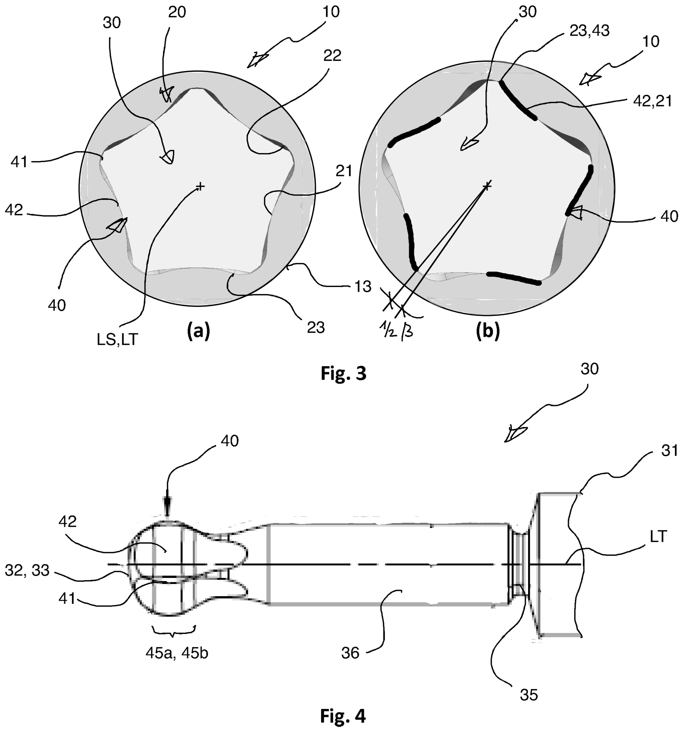

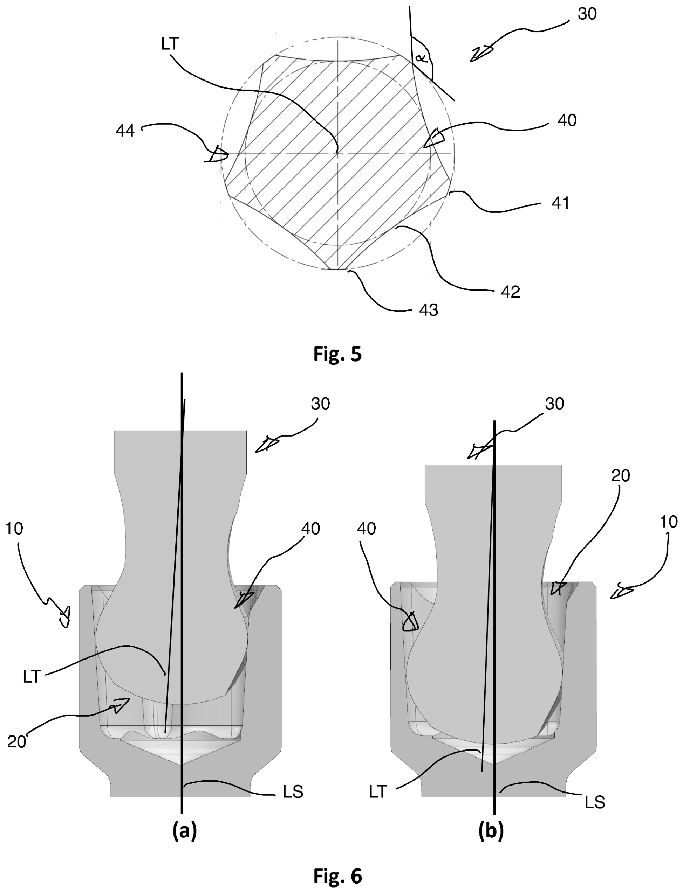

However and as described above, the transition between these sections may instead be a discontinuous transition. An example of a discontinuous transition along a profile of the engagement structure is shown in figure 5 for a fastening tool 30. Here, a point of transition 43 of a screw engaging interface 40 of the fastening tool 30 is discontinuous in terms of a change in slope. Due to this discontinuity, the point of transition 43 represents a corner along the profile of the screw engaging interface 40 as seen in a perpendicular cross-section in relation to the longitudinal axis LT of the dental fastening tool 30. In the engagement structure 44, this point of transition 43 of the profile results in a longitudinal edge (cf. figure 4).

At the point of transition 23, the transfer of torque from a fastening tool 30 to the dental screw 10 is most effective. The tangent to the profile of the tool engaging interface 20 at this point of transition 23 extends in a direction closest to a radial direction. As a result, a torque applied by means of a fastening tool results at this point along the engagement profile in the maximum force component perpendicular to the surface of the tool engaging interface 20 and the minimum force component parallel to this surface. Consequently, torque transfer is very efficient at this point between a dental fastening tool and the dental screw 10, whereas the amount of wear is relatively low. Thus, it is advantageous to transfer torque between a fastening tool and the dental screw 10 at and near the point of transition 23.

A dental fastening tool 30 for transferring torque to a dental screw 10 comprises an engagement structure 44 that corresponds to the engagement structure 24. Figure 5 shows an exemplary embodiment of such a dental fastening tool 30 with an engagement structure 44 in a perpendicular cross-section in relation to a longitudinal axis LT of this tool. The engagement structure 44 of the dental fastening tool 30 is part of a torque transfer zone 45b (cf. figure 4). Corresponding to the dental screw 10, the cross-sectional profile of the engagement structure 44 comprises convex sections 41 and concave sections 42. The convex sections 41 and the concave sections 42 merge or transition into each other at points of transition 43. Like the points of transition 23 of the torque transfer zone 25b of the dental screw 10, the point of transition 43 may be continuous or discontinuous points of transition 43. As already explained above, the point of transition 43 of the dental fasting tool 30 illustrated in figure 5 is a discontinuous point of transition.

Further, the concave section 42 is preferably curved. The convex section 41 may be curved and/or planer. In the exemplary embodiment illustrated in figure 5, the convex section 41 is curved and, more specifically, has a circular shape.

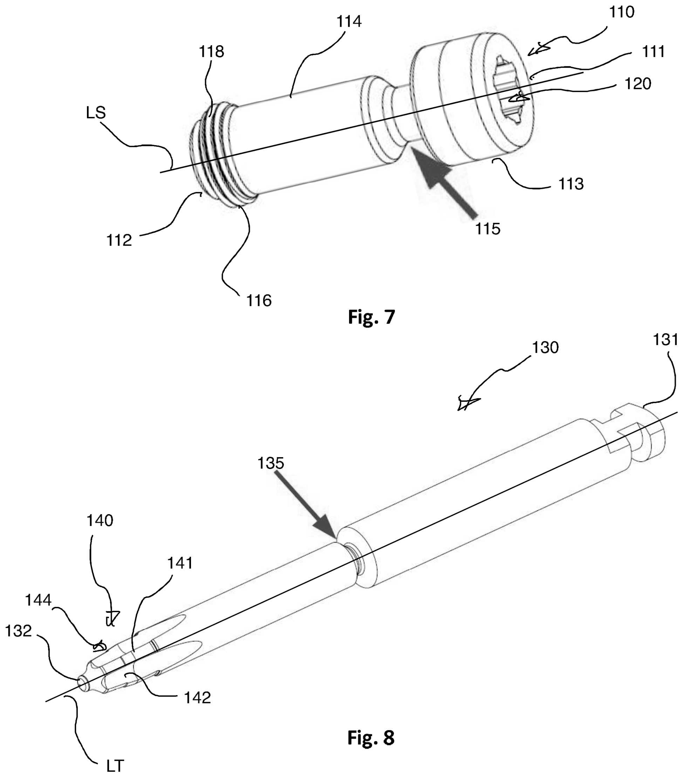

As described above, the angle at the point of transition 43 between a tangent to the convex section 41 and a tangent to the concave section 42 is preferably in a range of 90° to 180° or to 160°, preferably 95° to 150°. The closer this angle is to 90°, the more efficient is the torque transfer from the dental fastening tool 30 to the dental screw 10 at the point of transition 43. At 90°, the transfer of torque is basically performed in a circumferential direction. There is basically no force component acting in a direction parallel to the surface of the engagement structure 44 (which extends radially) and, as a result, wear of this surface caused by torque transfer can basically be prevented. For example, the dental fastening tools 130, 230 shown in figures 8 to 11 are designed for such an efficient torque transfer.

Figures 3 show a dental fastening tool 30 that has been inserted into a dental screw 10 (see figure 3a). The dental fastening tool 30 is then rotated about its longitudinal axis LT so that the engagement structure 44 of the dental fastening tool 30 is brought into engagement with the engagement structure 24 of the dental screw 10 (see figure 3b). As described above, this rotational play provides haptic feedback to a dental professional and facilitates insertion of the screw engaging interface 40 of the fastening tool 30 into the tool engaging interface 20 of the dental screw 10. The rotation of the screw engaging interface 40 within the tool engaging interface 20 about the aligned longitudinal axes LT and LS from the intermediate position in figure 3a to the engagement position in figure 3b is about 0.5 times **β**. As described above, the rotational play **β** between rotational engagement orientations, i. e. an engagement orientation for fastening and an engagement orientation for loosening is preferably 1° to 3.5° and preferably 1.5° to 3°.

Once in engagement, torque is transferred across the concave section 42 of the dental fastening tool 30 to the convex section 21 of the dental screw 10. This is schematically indicated in figure 3b by means of thick black lines. More specifically, torque is transferred along the profiles of the engagement structures 24, 44 between the transition points 23, 43 to intermediate positions, preferably the middle position, along the concave sections 42 of the fastening tool 30 and the convex sections 21 of the dental screw 10. In other words, there is essentially no transfer of torque along the profile in the region of the convex sections 41 of the fasting tool 30 and the convex sections 21 of the dental screw 10. This is due to a gap being present between the convex sections 41 and the concave sections 22 facing each other while being engaged state. As a result, no wear is caused in this area. Further, torque is provided in an efficient way at the transition point and its vicinity. Transfer of torque also takes place in an area further away from the outer wall of the dental screw 10, i. e. in an area with a higher wall thickness. Due to this and the torque transfer along the curved concave section 42 and the correspondingly curved convex section 21, stress concentrations are avoided. Consequently, these features also enable a more compact design of the dental screw.

Figure 4 illustrates a partial view of a dental fastening tool 30 in a longitudinal cross-section. The dental fastening tool 30 is also illustrated in the longitudinal cross-section in figures 6 referred to further below. As illustrated in figure 4, the torque transfer zone 45b of the screw engaging interface 40 is curved. More specifically, it is in particular at least partly a basically circular profile. Further, at the distal end 32, the dental fastening tool 30 preferably has a rounded tip 33. This geometric configuration allows to tilt the dental fastening tool 30 while transferring torque from the dental fastening tool 30 via the screw engaging interface 40 to the tool engaging interface 20 of the dental screw 10. This is, for example, illustrated in figures 6, where the longitudinal axis LS of the dental screw is inclined in relation to the longitudinal axis LT of the fastening tool 30. Further, above-described curved and rounded configuration of the screw engaging interface 40 prevents damage or scratches to the surface of the engagement structure 24, which may otherwise reduce the strength of the dental screw 10. Similar to the dental screw 10, the torque transfer zone 45b also acts as pick-up zone 45a as will be described in more detail further below.

The dental fastening tool of figure 4 also comprises proximally to the screw engaging interface 40 a torque limiting portion 35. Preferably, a shank 36 is arranged between the screw engaging interface 40 and the torque limiting portion 35 so that the dental fastening tool 30 does not break within the tool engaging interface 20 of the dental screw 10 during failure. This facilitates removal of the part of the dental fastening tool 30 comprising a portion that remains inside the tool engaging interface 20.

As already described above, the torque limiting portion 35 is configured to fail if a torque applied exceeds a predefined maximum value. Preferably, the torque limiting portion is configured to bear a maximum torque of 35 Ncm or less, and preferably in a range of 27.5 Ncm to 34 Ncm and more preferably in a range of 28 Ncm to 33 Ncm.

As illustrated in figure 4, the torque limiting portion 35 may be designed with a reduced cross-section so that the strength at this cross-section is reduced. In other words, the torque limiting portion 35 has the lowest section modules along the dental fastening tool 30. Consequently, the torque limiting portion 35 has a section modulus that is lower than the section modulus of the waisted portion adjacent to the screw engaging interface 40 and the shank 36.

As illustrated in figure 1, the convex sections 21 of the tool engaging interface 20 that form the tip of the longitudinal engagement protrusions 24 are chamfered at their proximal ends, i.e. at the entrance for the dental fastening tool to the tool engaging interface 20. The resulting chamfers 27 facilitate insertion of a dental fasting tool. On the one hand, they allow an initial insertion of a dental fastening tool without the engagement structure 44 of the dental fastening tool 30 being correctly aligned with the engagement structure 24 of the dental screw 10 in a rotational direction. On the other hand, the longitudinal axis LS of the dental screw 10 may initially have too much inclination in relation to the longitudinal axis LT of the fastening tool 30. Here, the chamfers 27 have a guiding function.

Further, the dental screw 10 in figure 1 comprises a tapered tool engaging interface 20. More specifically, the tool engaging interface 20 becomes narrower in a direction from the proximal end 11 to the distal end of the dental screw 10. In other words, the tool engaging interface 20 may have a substantially conical shape. Due to the tapered shape, the tool engaging interface 20 forms a pick-up zone 25a for picking-up and retaining a dental screw. In this exemplary embodiment of a dental screw and as illustrated in figures 1, 2, and 6, the pick-up zone 25a and the torque transfer zone 25b overlap each other.

The pick-up function will be explained in the following in more detail under reference to figures 6. As illustrated in figure 6a, a fastening tool 30 may engage the dental screw 10 by friction when the fastening tool 30 is inserted along the longitudinal axis LS into the recess of the tool engaging interface 20. Since the tool engaging interface 20 is tapered, the friction caused due to insertion of the fastening tool 30 enables the pickup function. More specifically, the dental screw 10 can be retained by the fastening tool 30.

As described above, the angle of the taper is preferably chosen in a range of 5° to 12°, in particular 6° to 9°, or may be substantially 7°. Alternatively, it may be in a range of 10° to 12°, or may be substantially 11°. These values allow to safely retain a dental screw and at the same time allow the connection between a fastening tool 30 and the dental screw 10 to be easily releasable. Thus, these angles also prevent unnecessary wear from occurring.

Besides the pick-up function, the taper of the tool engaging interface 20 also ensures optimum engagement between the engagement structures of the dental fastening tool 30 and the dental screw 10. This is schematically shown in figures 6a and 6b. In figure 6a, the dental fastening tool 30 has its initial size, i.e. it has not been subject to wear. As a result, engagement between the dental fastening tool 30 or more specifically the screw engaging interface 40 occurs on the proximal side of the tool engaging interface 20 of the dental screw 10.

Preferably, the dental fastening tool 30 is configured to be reusable and is preferably made of metal, in particular steel. As a result, wear occurs during continued use and reuse of this tool. Wear occurring at the engagement structure 44 of the dental fastening tool 30 results in a decrease of the size of this structure. However, since the tool engaging interface 20 of the dental screw 10 is tapered, the dental fastening tool 30 will simply be pushed further into the tool engaging interface 20. This achieves the same quality of engagement, which ensures an efficient transfer of torque. This is shown in figure 6b, where the dental fastening tool 30 that has been subject to wear is now engaging the tool engaging interface 20 of the dental screw 10 more distally.

Figure 7 illustrates another embodiment of a dental screw 110. As illustrated in figure 7, the dental screw 110 comprises a torque limiting portion 115 that is directly adjacent to the screw head 113. The skilled person will appreciate that such a torque limiting portion 115 may also be present along the screw shank 14 in the embodiment of a dental screw 10 illustrated in figure 1, either at the same position or at a different position along the screw shank 14.

Similar to the torque limiting portion 35 described in relation to the dental fastening tool 30, the torque limiting portion 115 has the smallest section modulus along the part of the body of the dental screw 110 that transfers torque, i.e. the part of the body that is subject to increasing shear stresses and tensile stresses during fastening of the dental screw 110.

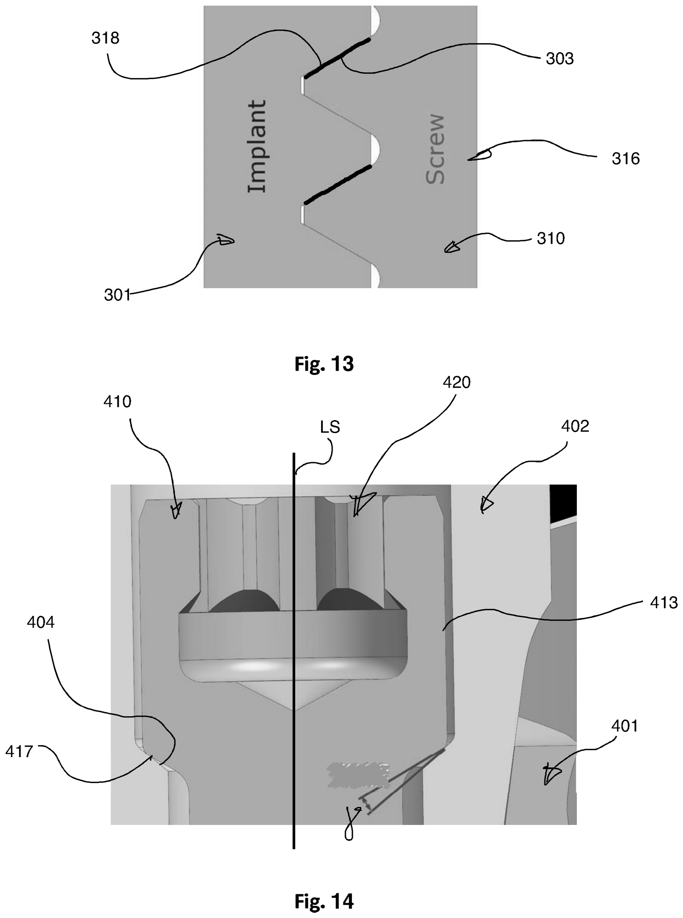

The torque limiting portion 115 is part of and arranged along the screw shank 114. At the end of the screw shank 114, i. e. at the distal end 112 of the dental screw 110, is a threaded portion 116. The threaded portion 116 is for engaging a dental component such as a dental implant 401 or an abutment 402 (cf. figure 14). For example, the dental screw 110 may be used to attach an abutment 402 to a dental implant 401.

As already defined above, the minimum torque that the torque limiting portion 115 of the dental screw 110 should withstand and transfer is preferably 26 Ncm. When exceeding the maximum torque value, the torque limiting portion 115 should particularly prevent failure of any part of the dental screw 110 distally of the torque limiting portion 115, in particular a failure of the threaded portion 116. Controlling the failure of the dental screw in this manner ensures that the dental component, which is in engagement with the dental screw 110, remains intact. This is particularly advantageous in case of the dental component been a dental implant that is anchored within the bone tissue of a patient since a revision of the implant is prevented.

Figure 8 illustrates another embodiment of a dental fastening tool 130. The screw engaging interface 140 at the distal end 132 of the dental fastening tool 130 also comprises convex sections 141 and concave sections 142, which form a circumferential engagement structure 144. The dental fastening tool 130 is configured for a dental screw preferably comprising an engagement structure with a corresponding number of convex and concave sections. As described above, the convex sections may also be at least partially planar.

Nonetheless, the dental fastening tool 130 particularly differs from the dental fastening tool 30 of the previous figures in terms of the screw engaging interface 140. In particular, the screw engaging interface comprises four of each of the convex and concave sections instead of five.

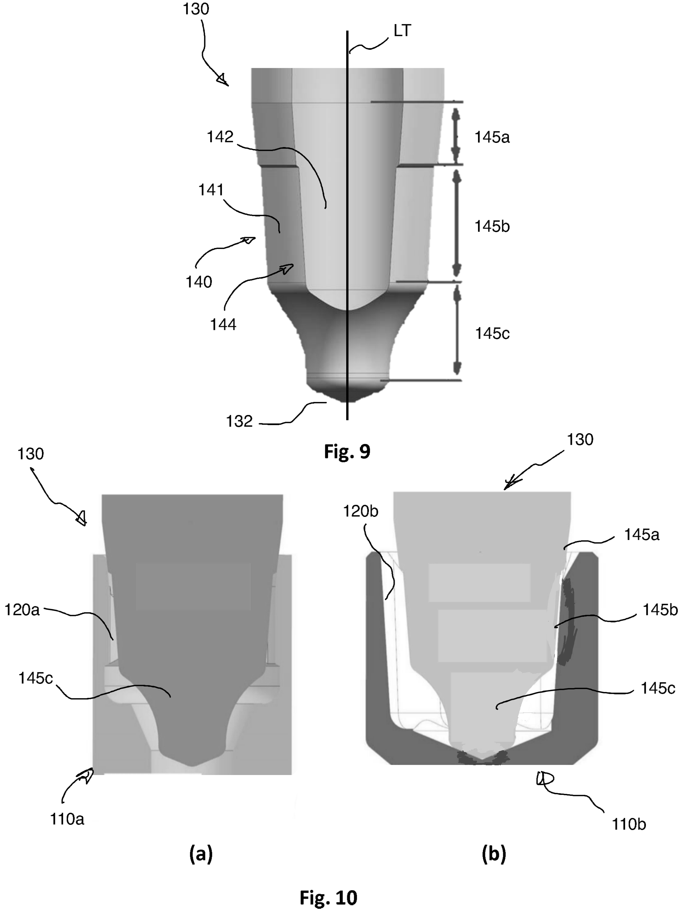

Turning to figure 9, the screw engaging interface 140 of the dental fastening tool 130 also differs in relation to the previous embodiments in that the pick-up zone 145a is separate from the torque transfer zone 145b. More specifically, the torque transfer zone 145b is located adjacent and distally to the pick-up zone 145a. In other words, the pick-up zone 145a does not overlap with the torque transfer zone 145b. The pick-up zone 145a basically has a conical shape for engagement with a corresponding pick-up zone of a dental screw by friction to facilitate handling of a dental screw as has already been described above.

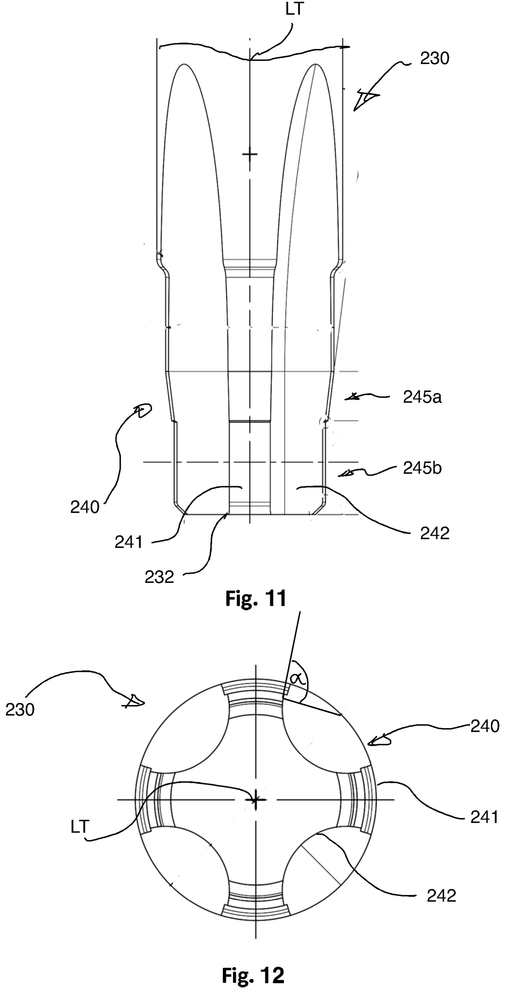

Although the torque transfer zone 145b of the dental fastening tool 130 of figure 9 has a concave shape similar to the pick-up zone 145a, it may alternatively have a cylindrical form as shown in another embodiment of a dental fastening tool 230 illustrated in figures 11 and 12 and described further down below.

In addition to the pick-up zone 145a and the torque transfer zone 145b, the dental fastening tool 130 comprises adjacent to its distal end 132 a verification zone 145c. On the one hand, this verification zone 145c has the function to prevent engagement with the tool engaging interface 120b of a dental screw 110b that does not comprise a verification zone with a corresponding shape (cf. figure 10b). Further, a dental screw 110b without a corresponding verification zone may also prevent engagement of a pick-up zone 145a since it will not be possible to insert the dental fastening tool 130 far enough into the tool engagement interface of this dental screw 110b for bringing these zones into engagement.

On the other hand, if a dental screw 110a comprises a corresponding verification zone such as the dental screw 110a in figure 10a, the dental fastening tool 130 is able to engage the tool engaging interface 120a of this screw.

Figures 11 and 12 illustrate yet another embodiment of a dental fastening tool 230 with a screw engagement interface 240 similar to the screw engaging interface 140 of the previous embodiment. However, in difference to the previous embodiment, the dental fastening tool 230 does not comprise a verification zone. Instead, the screw engaging interface 240 may only comprise a pick-up zone 245a and a torque transfer zone 245b. Although in the embodiment of a dental fastening tool 230 shown in figure 11 and in the embodiment of a dental fastening tool 130 shown in figure 10 the torque transfer zones 145b, 245b are located adjacent and distally to the pickup zones 145a and 245a, the skilled person will appreciate that this sequence may well be reversed. Thus, the pick-up zones 145a, 245a may be located distally to the torque transfer zones 145b, 245b. Further, the pick-up zones 145a, 245a and the torque transfer zones 145b, 245b do not have to be adjacent to each other but may be spaced apart from each other.

For a cost-efficient production, the concave sections 242 are machined in a proximal direction beyond the screw engaging interface 240. As a result, the pick-up zone 245a basically has a conical shape that is interrupted by concave sections. However, this is functionally not necessary although it may provide a more defined friction between the pick-up zone 245a and a corresponding dental screw. The same applies to the dental fastening tools of the previous embodiments.

Figure 12 illustrates a frontal view of the dental fastening tool 230 as seen from the distal end 232. As illustrated in this figure, the screw engaging interface 240 comprises four convex sections 241 alternating with four concave sections 242. As already noted in relation to the previous embodiments, the convex sections 241 may instead be planar sections. In this exemplary embodiment, the convex sections 241 are curved and have in particular a circular shape as seen in a perpendicular cross-section in relation to the longitudinal tool axis LT.

As shown by way of example in figure 12, the angle **α** between a tangent to the convex sections and a tangent to the concave sections is at least close to the optimal angle of approximately 90 °for transfer as explained above. In other words, the angle **α** is in above-noted lower range. Thus, the configuration of the torque transfer zone 245b of the screw engaging interface 240 provides for an efficient transfer of torque and is less prone to wear during continued use and reuse. For keeping wear of the dental fastening tools of this disclosure low, at least the screw engaging interfaces and preferably the fastening tool is made of metal, in particular steel.

In difference to the dental fastening tool 30 of the previous figures, the screw engaging interface 240 is only engageable with the tool engaging interface of a dental screw if the longitudinal tool axis LT and the longitudinal axis are basically in alignment. As described above, this alignment may be facilitated by providing chamfers at the entrance to the tool engaging interface of a dental screw.

Figure 13 is a schematic longitudinal cross-section of a threaded engagement between a dental component, in particular a dental implant 301, and a dental screw 310. Naturally, this configuration is applicable for any combination of a dental component and a dental screw.

In a tightened state, a distal flank 303 of the dental implant's thread is in tight contact with a proximal flank 318 of the dental screws threaded portion 316. Thus, the force provided by tightening the dental screw 310 to fasten another dental component to the dental implant 301 is transferred as contact force between the thread flanks 303 and 318.

In the configuration of this disclosure, the threaded hole of the implant 301 and the outer thread of the dental screw 310 are dimensioned so that the overlap of the dental flanks, indicated in figure 13 as black continuous lines, is at least 70% of the length of each of the thread flanks in a radial direction. The length of the thread flanks in a radial direction is defined as the length between the tip of a thread and its root that allows for transfer of forces in a longitudinal direction of the threaded screw shaft of the dental screw or threaded hole of the dental component. For comparison, normal threads such as threads according to the ISO-standard only have an overlap of at least 60%.

Further, the overlap of the threaded connection according to this embodiment is up to 90%, 93%, and preferably 96%. Nonetheless, it is particularly the difference in minimum overlap of 10% in comparison to ISO-threads that allows for a significantly improved strength of the thread. This result enables a more compact design of the dental screw 310 since the transfer of compressive forces within this threaded connection is more effective.

Figure 14 shows a partial cross-sectional view in a longitudinal direction along the longitudinal screw axis LS. It illustrates an abutment 402 that is fastened to a dental implant 401 via a dental screw 410. As described above, the torque limiting portion of a dental screw according to the present disclosure is dimensioned to transfer at least a minimum amount of torque. Further, the dental screw is configured for a controlled failure at torque values that exceed a maximum torque value.

When fastening the dental screw 410 of figure 14 to the dental implant 401, a support surface 417 of the screw head 413 at the distal end of the screw head 113 is pressed against a screw seat 404 of the dental abutment 402. As a result of the contact established between the support surface 417 and the screw seat 404, a part of the torque transferred from a dental fastening tool (not shown) via the tool engaging interface 420 of the dental screw 410 is transferred to the abutment 402.

As also shown in figure 14, the screw seat 404 and the support surface 417 have a conical shape. The conical shape of the screw seat 404 and the conical shape of the support surface 417 have cone angles that differ from each other. Preferably, the cone angle of the support surface 417 is larger than the cone angle of the screw seat 404. However, a configuration, in which the cone angles are dimensioned vice versa is alternatively possible. The difference between the cone angles of the support surface 417 and the screw seat 404 is indicated as γ in figure 14.

Due to this angle γ, a contact between the support surface 417 and the screw seat 404 is essentially established by a line contact. This is on the one hand advantageous since such a line contact causes less torque to be transferred from the dental screw 410 to the abutment 402. On the other hand, the torque transferred and not used to tighten the threaded connections can be more accurately determined. Both advantages allow for a more predictable behavior of a torque limiting portion of the dental screw 410. In other words, this configuration renders the failure of the dental screw 410 at the torque limiting portion more reliable and, thus, may contribute to a more compact design of the dental screw 410.

As previously described, the gap created due to the difference γ between the cone angles may at least partially be closed at the end of fastening process of the dental screw 410. This closure of the gap is caused by deformation of the screw seat 404 and the support surface 417 and further decreases the amount of torque that is transferred towards the threaded portion of the dental screw 410 in relation to the amount of torque that is transferred to the abutment 402. This change in transfer ratio has a dampening effect during the application of torque and enables a more precise adjustment of the maximum torque at the end of fastening the threaded connection between the dental screw 410 and the dental implant 401. Thus, these features of the screw seat 404 and the support surface 417 and their configuration in a dental assembly also enable a more compact design for the dental screw 410.

### REFERENCE SIGNS

The following lists the last two digits of the reference signs used in the description and the drawings. Throughout the drawings these digits refer to features that have the same or an equivalent function and/or structure. The preceding digit represents the embodiment.
- 1: dental implant
- 2: abutment
- 3: flank of threaded hole
- 4: screw seat
- 10: dental screw
- 11: proximal end
- 12: distal end
- 13: screw head
- 14: screw shank
- 15: torque limiting portion
- 16: threaded portion
- 17: support surface
- 18: flank of threaded portion
- 20: tool engaging interface
- 21: convex section
- 22: concave section
- 23: point of transition
- 24: engagement structure of dental screw
- 25a: pick-up zone
- 25b: torque transfer zone
- 25c: verification zone
- 26: circle with maximum inner diameter
- 27: chamfer
- 30: fastening tool
- 31: proximal end
- 32: distal end
- 33: rounded tip
- 35: torque limiting portion
- 36: shank
- 40: screw engaging interface
- 41: convex section
- 42: concave section
- 44: engagement structure of fastening tool
- 45a: pick-up zone
- 45b: torque transfer zone
- 45c: verification zone

- LS: longitudinal axis of screw
- LT: longitudinal axis of fastening tool
- α: angle at point of transition between tangent to convex section and tangent to concave section
- β: rotational play between dental screw and fastening tool
- γ: difference in cone angle between screw seat and support surface of screw head

## Claims

1. A dental screw (10) for fixing dental components (1, 2) to each other, the dental screw having a proximal end (11), a distal end (12), and a longitudinal axis (LS), wherein the dental screw further comprises a screw head (13) including a tool engaging interface (20) for engaging a fastening tool (30) at the proximal end and distally to the screw head a screw shank (14), the screw shank comprising a torque limiting portion (15) and distally to the torque limiting portion a threaded portion (16) for engaging a dental component (1, 2), wherein the dental screw is configured to transfer a minimum torque of 26 Ncm and the torque limiting portion is configured to limit the torque transferable to a maximum torque for preventing damage to the threaded portion.

2. The dental screw (10) according to claim 1, wherein the torque limiting portion (15) has the smallest section modulus along the shank.

3. The dental screw (10) according to claim 1 or 2, wherein the maximum torque transferable at the torque limiting portion (15) is 38 Ncm, 36 Ncm, 34 Ncm, or 32 Ncm, in particular wherein the torque limiting portion (15) is configured to withstand a maximum tensile force in a range of 300 N to 500 N and preferably in a range of 350 N to 450 N.

4. The dental screw (10) according to any one of the preceding claims, wherein the tool engaging interface (20) includes a torque transfer zone (25b) having a cross-section perpendicular to the longitudinal axis (LS) comprising a profile with four or five convex and/or planar sections (21) alternating with four or five concave sections (22), respectively, in particular wherein the convex sections (21) and concave sections (22) form longitudinal engagement protrusions (24) radially extending from a circle with a maximum inner diameter (26) towards the longitudinal axis (LS), the engagement protrusions preferably having chamfers (27) at their proximal ends.

5. The dental screw (10) according to any one of the preceding claims, wherein the tool engaging interface (20) includes a pick-up zone (25a) that tapers in a direction from the proximal end (11) to the distal end (12) of the dental screw, the pick-up zone preferably being located adjacent, proximally to, and/or at least partially along the torque transfer zone (25b).

6. The dental screw (10) according to any one of the preceding claims, wherein the tool engaging interface (20) further includes a verification zone (25c) for verifying the type of the dental screw (10), the verification zone preferably being located at the distal end of the tool engaging interface.

7. A dental fastening tool (30), the dental fastening tool having a proximal end (31), a distal end (32), and a longitudinal axis (LT), wherein the dental fastening tool comprises a screw engaging interface (40) at the distal end for engaging a dental screw (10), in particular a dental screw according to any one of the preceding claims, and proximally to the screw engaging interface a torque limiting portion (35), the torque limiting portion being configured to limit the torque transferable to a maximum torque for preventing damage to the screw engaging interface.

8. The dental fastening tool according to claim 7, wherein the torque limiting portion (35) is configured to bear a maximum torque of 35 Ncm or less, preferably in a range of 27.5 Ncm to 34 Ncm and more preferably in a range of 28 Ncm to 33 Ncm.

9. The dental fastening tool (30) according to claim 7 or 8, wherein
the screw engaging interface (40) includes a torque transfer zone (45b) with a cross-section perpendicular to the longitudinal axis (LT) comprising four or five convex and/or planar sections (41) alternating with four or five concave sections (42), respectively, and
the convex and/or planar sections (21) and concave sections (22) form an engagement structure (44) about the longitudinal axis (LT) along the dental fastening tool, the engagement structure preferably having a curved profile in a cross-section along the longitudinal axis (LT),in particular wherein the convex and/or planar sections (41) and concave sections (42) are connected at points of transition (43), and at each point of transition an angle (**α**) between a tangent to the profile of the concave section and a tangent to the convex section or the planar section is in a range of 90° to 180° or to 160°, preferably 95° to 150°.

10. The dental fastening tool (30) according to any one of claims 7 to 9, wherein the screw engaging interface (40) includes a pick-up zone (45a) for holding a dental screw (10), the pick-up zone preferably being located adjacent, proximally to, and/or at least partially along the torque transfer zone (45b).

11. The dental fastening tool (30) according to any one of claims 7 to 10, wherein the screw engaging interface (40) further includes a verification zone (45c) for verifying the type of a dental screw (10) when engaging said dental screw, the verification zone preferably being located at the distal end of the tool engaging interface.

12. A dental fastening set comprising:
a dental screw (10), in particular a dental screw according to any one of claims 1 to 6, and
a dental fastening tool (30), in particular a dental fastening tool according to any one of claims 7 to 11,
wherein the dental screw and the dental fastening tool each comprise a torque limiting portion (15, 35), wherein the torque limiting portion (15) of the dental screw is configured to bear a maximum torque that is higher than the maximum torque that is transferable by the torque limiting portion (35) of the dental fastening tool.

13. The dental fastening set according to claim 12,
the dental screw (10) comprising a tool engaging interface (20), wherein the tool engaging interface includes a torque transfer zone (25b) with a cross-section comprising convex and/or planar sections (21) alternating with concave sections (22); and
the dental fastening tool (30) comprising a screw engaging interface (40), wherein the screw engaging interface includes a torque transfer zone (45b) with a cross-section comprising convex and/or planar sections (41) alternating with concave sections (42),
wherein in an engaged state of the dental screw and the dental fastening tool for transferring torque, at least an intermediate section of each of the convex and/or planar sections of the dental screw faces a profile of a concave section (22) of the tool engaging interface (20) at a distance.

14. The dental fastening set according to claim 12 or 13, wherein the torque transfer zone (25b) of the dental screw (10) and the torque transfer zone (45b) of the dental fastening tool (30) have a rotational play when being engaged with each other, in particular a play in a range of 1° to 3.5° and preferably 1.5° to 3°.

15. The dental fastening set according to any one of claims 12 to 14, wherein the dental screw (10) and the dental fastening tool (30) each comprise a pick-up zone (25a, 45a) configured to engage each other by friction.

16. The dental fastening set according to any one of claims 12 to 15, wherein the dental screw (10) and the dental fastening tool (30) each comprise a verification zone (25c, 45c) configured to allow engagement of the torque transfer zone (45b) of the dental fastening tool (30) and the torque transfer zone (25b) of the dental screw (10) when the dental fastening set is fully engaged.

17. A dental restoration support set comprising a dental screw according to any one of claims 1 to 6, and a dental component (1; 2), wherein the dental component is in particular a dental implant (3), an abutment (2), and/or an artificial tooth.
